# EUROPÄISCHE PATENTSCHRIFT

(11) **EP 3 388 030 B1**
(45) Veröffentlichungstag und Bekanntmachung des Hinweises auf die Patenterteilung: **04.03.2020**
(21) Anmeldenummer: 17166465.9
(22) Anmeldetag: 13.04.2017
(51) Int. Cl.: A61F 2/36, A61F 2/38, A61F 2/40, A61F 2/30

(54) **SPACER ALS TEMPORÄRER GELENKPROTHESENERSATZ ZUR ABGABE EINES MEDIKAMENTENWIRKSTOFFS**
SPACER AS A TEMPORARY JOINT PROSTHESIS FOR DISPENSING A MEDICAL AGENT
ESPACEUR EN TANT QUE REMPLACEMENT TEMPORAIRE DE PROTHÈSE D'ARTICULATION POUR DÉLIVRER UN MÉDICAMENT ACTIF

(43) Veröffentlichungstag der Anmeldung: 17.10.2018
(73) Patentinhaber: Waldemar Link GmbH & Co. KG, 22339 Hamburg (DE)
(72) Erfinder: Link, Helmut D., 22397 Hamburg (DE)
(74) Vertreter: Glawe, Delfs, Moll

(56) Entgegenhaltungen:
- EP-A1- 2 823 790
- EP-A1- 2 853 242
- WO-A1-2016/071938
- US-A- 5 123 927
- US-A1- 2010 102 484
- US-B1- 6 361 731

## Beschreibung

Die Erfindung betrifft einen Spacer als temporären Gelenkersatz für eine Endoprothese. Genauer gesagt geht es um einen Spacer für eine Gelenkendoprothese, umfassend einen Hauptkörper zur Befestigung an einem Knochen und ein Gelenkstück an einem Ende des Hauptkörpers, wobei der Hauptkörper ein Material umfasst, das zur Abgabe eines Medikamentenwirkstoffs an umliegendes Gewebe ausgebildet ist.

Bei Patienten mit einer Gelenkendoprothese kann es postoperativ oder im Laufe der Zeit dazu kommen, dass Infektionen in dem die Prothese umgebenden Gewebe auftreten. Das ist insbesondere dann problematisch, wenn das von der Infektion betroffene Gewebe schlecht zugänglich ist, insbesondere sich im Inneren des Knochens befindet, in dem die Gelenkendoprothese implantiert ist. Zur Behandlung ist es dann häufig erforderlich, die Gelenkendoprothese zumindest teilweise zu entfernen, um so vor dem Einsetzen einer Revisionsprothese eine Behandlung des infizierten Gewebes zu ermöglichen. Hierbei treten zwei Schwierigkeiten auf: zum einen muss der erforderliche Medikamentenwirkstoff an die betroffene Stelle, welche häufig im Knochen liegt, gebracht werden, und zum anderen eine Verkürzung des Gelenks, wie sie an sich durch das Entfernen des Teils der Gelenkendoprothese eintritt, möglichst verhindert werden, um eine Degeneration des Gelenks (insbesondere Sehnen und Bänder) zu vermeiden. Zu diesem Zweck ist es bekannt, sogenannte Spacer temporär an die Stelle des entfernten Teils der Gelenkendoprothese zu platzieren. Dieser Spacer füllt nicht nur das Leervolumen auf und verhindert durch seine Anwesenheit eine unerwünschte Verkürzung des umliegenden Gewebes, sondern ist auch so beschaffen, dass er einen zur Behandlung der Entzündung geeigneten Medikamentenwirkstoff abgibt. Derartige Spacer sind an sich bekannt, wie zum Beispiel aus der US 2010/0102484 A1, die auch den nächstliegenden Stand der Technik darstellt.

Während der zur Bekämpfung der Entzündung erforderlichen Zeit sollte der Patient mobilisiert werden, wenn auch nicht unbedingt mit vollem Gewicht. Das ist wichtig, um die Beweglichkeit zu erhalten. Es hat sich gezeigt, dass bei dieser Mobilisierung durch die Interaktion zwischen Spacer und dem verbleibenden Teil der Gelenkendoprothese Abrieb entsteht, wodurch nicht geringe Mengen körperfremden Materials, nämlich des Substratmaterials des Spacers als Abrieb, in das umliegende Gewebe gelangen. Dies kann ausgesprochen negative Langzeitfolgen für den Patienten nach sich ziehen, einschließlich eines vorzeitigen Ausfalls der Revisionsprothese.
Der Erfindung liegt die Aufgabe zugrunde, einen verbesserten Spacer zur Vermeidung dieser negativen Langzeitfolgen zu schaffen.
Die erfindungsgemäße Lösung liegt in den Merkmalen des unabhängigen Anspruchs. Vorteilhafte Weiterbildungen sind Gegenstand der abhängigen Ansprüche.
Bei einem Spacer für eine Gelenkendoprothese, umfassend einen Hauptkörper zur Befestigung an einem Knochen und ein Gelenkstück an einem Ende des Hauptkörpers, wobei der Hauptkörper ein Material umfasst, das zur Abgabe eines Medikamentenwirkstoffs an umliegendes Gewebe ausgebildet ist, und das Gelenkstück zur beweglichen Interaktion mit einer Gelenkstückaufnahme ausgebildet ist, ist erfindungsgemäß vorgesehen eine gesonderte Überkappung des Gelenkstücks, die aus einem verschleißfesten Material besteht, und verdrehfest auf dem Gelenkkopf gehaltert ist.

Der Erfindung liegt die Erkenntnis zugrunde, dass mit dem Dazwischenschalten eines weiteren Elements, nämlich der Überkappung, eine Verringerung des Abriebs erreicht werden kann. Dies ist insofern überraschend, da mit der Schaffung des zusätzlichen Elements die Anzahl der Reibflächen an sich verdoppelt wird (nämlich einmal zwischen Gelenkstück und Überkappung und zum anderen zwischen der Überkappung und dem Gegenpart, nämlich der Gelenkstückaufnahme). Die Erfindung hat erkannt, dass dies durch eine Kombination zweier Maßnahmen verhindert werden kann, nämlich indem die Überkappung selbst aus einem an sich verschleißfesten Material besteht, und zum anderen eine verdrehfeste Befestigung gegenüber dem Gelenkkopf vorgesehen ist. Damit wird die negative Wirkung der zweiten Reibungsfläche eliminiert. Dank der verschleißfesten Gestaltung der Überkappung fällt bei der Artikulation kein Abrieb des Spacermaterials mehr an, da insoweit die Verhältnisse ähnlich sind wie bei der regulären Gelenkendoprothese.

Damit kann auch während der Zeit, während der der Spacer implantiert ist, die Mobilität des Patienten durch regelmäßiges Bewegungstraining gefördert werden unter Vermeidung des für den Patienten beträchtlichen Risikos einer Schädigung durch Spacer-Abrieb. Die Erfindung beseitigt somit den bisher bestehenden Gegensatz zwischen dem Erfordernis nach Mobilität einerseits und dem damit einhergehenden Risiko von Komplikationen (durch Abrieb) andererseits.

Unter einem Spacer wird ein zur temporären Implantation vorgesehenes Implantat verstanden, welches im Wesentlichen zur Abgabe von Medikamentenwirkstoff und zur Verhinderung der Kontraktion des betroffenen Gelenks und dessen zugeordneten Funktionseinheiten (Muskeln, Sehnen etc.) dient.

Unter dem Gelenkstück und der Gelenkstückaufnahme werden die beiden beweglich miteinander zusammenwirkenden Komponenten eines Gelenks, insbesondere eines Beugegelenks, verstanden.

Vorzugsweise handelt es sich bei dem verschleißfesten Material um ein gleitgünstiges Material. Insbesondere sind hierfür Edelstahl, Kobalt-Chrom-Molybdän, Titan, hochfestes Kunststoffmaterial (Polyetheretherketon - PEEK) oder andere biokompatible Materialien geeignet. Mit diesem metallischen Material wird auf einfache Weise eine reibungsarme und verschleißfeste Ausführung der Überkappung erreicht. Im Grunde beruht dies auf der Erkenntnis, dass somit eine ähnliche Materialauswahl getroffen wird, wie bei der zur Dauerversorgung vorgesehenen Prothese an sich. Zweckmäßigerweise ist das verschleißfeste Material biokompatibel. So wird wirkungsvoll dem Risiko begegnet, dass sich die Überkappung wegen ihres Materials negativ auf den Patienten auswirken könnte.

Mit Vorteil ist die Überkappung an ihrer der Gelenkstückaufnahme zugewandten Außenseite geglättet. Auf diese Weise wird eine reibungsarme Interaktion mit der Gelenkstückaufnahme erreicht. Die Gelenkfunktion kann damit weitgehend wieder erreicht werden. Dies begünstigt nicht nur die Mobilität, sondern die reibungsarme Ausführung vermindert auch die Gefahr der Bildung von weiterem Abrieb.

Die Gelenkstückaufnahme kann natürlich sein (z.B. die Gelenkpfanne am Acetabulum im Falle eines Spacers für die Femurkomponente einer Hüftprothese) oder künstlich (z.B. ein Gelenkpfannenimplantat). Es handelt sich hierbei um die Komponente, die funktional mit dem Gelenkstück des Spacers zusammenwirkt zur Bildung eines Gelenks.

Mit Vorteil sind Verankerungselemente vorgesehen zur verdrehfesten Halterung der Überkappung auf dem Gelenkkopf. Damit kann eine robuste formschlüssige Verbindung erreicht werden. Zwingend ist dies aber nicht. Es kann auch vorgesehen sein, dass die gewünschte Verdrehfestigkeit auf andere Weise erreicht wird, z.B. durch Klemmung.

Zweckmäßigerweise weist die Überkappung eine halbkugelförmige Gestalt auf. Dies beruht auf der Erkenntnis, dass damit eine wirksame Übermantelung des Gelenkstücks erreicht werden kann. Die halbkugelförmige Ausführung ermöglicht ein Maximum an Beweglichkeit bei weiterhin noch einfacher Montage der Überkappung auf dem Gelenkkopf. Dies hat sich insoweit für die Erfindung als optimal herausgestellt. Es soll aber nicht ausgeschlossen sein, dass zur Gewährleistung einer höheren Befestigungssicherheit im montierten Zustand die Überkappung geringfügig über den Äquator der halbkugelförmigen Gestaltung hinausreicht, vorzugsweise maximal 20°, weiter vorzugsweise maximal 5°. Damit ist im Regelfall noch eine leichte Montage möglich unter Nutzung der elastischen Eigenschaften des für die Überkappung verwendeten Materials. Weiter gibt dies einen Vorteil einer erhöhten Sicherheit gegenüber einer unerwünschten Dislokation der Überkappung von dem Gelenkkopf. Es kann mit Vorteil auch eine eigenständige Dislokationssicherung an der Überkappung vorgesehen sein, die ein Ablösen von dem Gelenckopf verhindert.

Die Überkappung kann beispielsweise eine geschlossene, homogene Oberfläche aufweisen. Erfindungsgemäß ist aber vorgesehen, dass die Überkappung eine Mehrzahl von Durchgangsöffnungen aufweist. Damit wird eine bessere Versorgung des umliegenden Gewebes mit dem Medikamentenwirkstoff auch im Bereich des Gelenkstücks erreicht. Die Durchgangsöffnungen sind dabei in einem nicht-lasttragenden Bereich der Überkappung angeordnet. Generell ist es zweckmäßig, den Medikamentwirksttoff auch im Bereich des Gelenkstücks vorzusehen. Zweckmäßigerweise ist eine Rotationssicherung für die Überkappung vorgesehen. Sie dient dazu, ein Verdrehen der Überkappung relativ zu dem Gelenkkopf zu verhindern. Eine solche Verdrehung ist für die Funktionalität des Gelenkstücks, auch im Zusammenwirken mit der Gelenkstückaufnahme, zwar nicht von entscheidendem Nachteil. Jedoch kann eine solche relative Verdrehung der Überkappung zu dem Gelenkkopf die Gefahr mit sich bringen, dass dort in erhöhtem Maße Abrieb generiert würde. Dieser Gefahr lässt sich durch besagte Rotationssicherung wirkungsvoll begegnen.
Bei einer vorteilhaften Ausführungsform weist der Hauptkörper eine solche Gestalt auf, dass er das Ende eines Knochens umgreift. Damit kann eine Anwendung an einem flächenmäßig ausgedehnten Gelenk erreicht werden. Ein Beispiel hierfür ist ein Spacer für die Femurkomponente einer Knieprothese, wobei die Überkappung kondylenartig konvex geformt ist. Durch das Umgreifen ergibt sich direkt eine hohe Verdrehsicherheit gebende Befestigung auf dem Knochen. Alternativ oder zusätzlich kann vorgesehen sein, dass der Hauptkörper einen Verankerungsschaft umfasst. Mit diesem kann der Spacer mit seinem Hauptkörper in dem Knochen verankert werden. Dies erhöht die Befestigungssicherheit. Zweckmäßigerweise ist der Verankerungsschaft zum Einsetzen in einen Hohlraum des Knochens ausgebildet, bspw. für die Tibiakomponente einer Knieprothese oder die Femurkomponente einer Hüftprothese.

Vorzugsweise ist eine Tragstruktur im Schaft vorgesehen. Sie verleiht dem Schaft eine höhere mechanische Festigkeit. Dies ermöglicht es, für den Körper des Schafts an sich ein Material vorzusehen, dessen Eigenschaften allein auf die Abgabe des Medikamentenwirkstoffs optimiert sind; auf dessen mechanische Eigenschaften, insbesondere Festigkeit, kommt es hierbei nicht entscheidend an, da eine ausreichende Festigkeit bereits durch die Tragstruktur im Inneren gewährleistet ist. Mit Vorteil ist die Tragstruktur mit einer Fortsetzung versehen, die bis zur Überkappung am Gelenkkopf reicht. Auf diese Weise wird eine zusätzliche Fixierung der Überkappung erreicht. Weiter wird eine nahtlose Einleitung der über das Gelenk übertragenen Kräfte in die Tragstruktur und damit den Schaft gewährleistet.

Es sei angemerkt, dass die Erfindung nicht auf die Anwendung am Hüftgelenk beschränkt ist, sondern auch bei Schulter-, Ellbogen sowie Kniegelenken und generell bei an sich beliebigen Gelenken mit Artikulation einsetzbar ist.

Bei einer Variante der Erfindung ist die Überkappung nicht halbkugelförmig, sondern als ein konvexes Kugeloberflächensegment ausgebildet. Es umfasst daher das Gelenkstück nicht vollständig. Es ist an einer vorbestimmten Position auf dem Gelenkkopf angeordnet, und zwar vorzugsweise an einer solchen Stelle, wo (bei stehendem Patienten) die Hauptlast von der Gelenkstückaufnahme auf den Spacer übertragen wird. Funktional wird damit wie bei dem ersten Ausführungsbeispiel ein Schutz gegenüber verschleißbedingtem Abrieb erreicht.

Es kann in einer weiteren Ausführungsform, die ggf. unabhängigen Schutz verdient, auch vorgesehen sein, dass für die Überkappung ein Vollmaterialstück aus verschleißfestem Material verwendet wird, beispielsweise ein Metallkopf statt des Gelenkstücks mit Überkappung (in der Ausführung als Voll- oder Halbkopf). Vorzugsweise ist ein solcher Vollmaterialkopf verbunden mit der Tragstruktur, wodurch ein besonders robuster Spacer geschaffen wird (letzteres ist insbesondere von Vorteil zur Anwendung bei adipösen Patienten).

Die Erfindung wird nachfolgend unter Bezugnahme auf die beigefügte Zeichnung anhand eines Ausführungsbeispiels näher erläutert. Es zeigen:
- Fig. 1: eine perspektivische Ansicht eines ersten Ausführungsbeispiels des Spacers;
- Fig. 2: eine Ansicht des Spacers von Fig. 1 mit abgesetzter Überkappung;
- Fig. 3a, b: verschiedene Ansichten zu Varianten der Überkappung;
- Fig. 4: eine perspektivische Ansicht eines zweiten Ausführungsbeispiels des Spacers;
- Fig. 5: eine perspektivische Ansicht eines dritten Ausführungsbeispiels; und
- Fig. 6: eine Explosionsansicht zu dem dritten Ausführungsbeispiel gemäß Fig. 5.

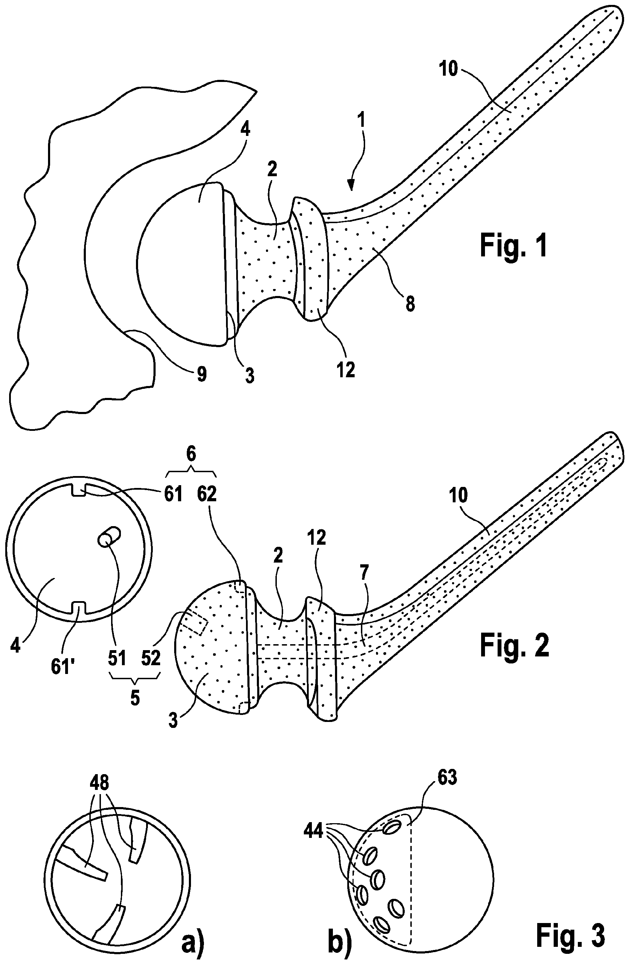

Ein erstes Ausführungsbeispiel für einen erfindungsgemäßen Spacer wird unter Bezugnahme auf die Figuren 1 und 2 erläutert. Der Spacer umfasst als Hauptkörper 1 einen langgestreckten Schaft 10 mit einem Hals 2. Der Schaft 10 ist von langgestreckter Gestalt und weist einen ovalen bis rechteckförmigen Querschnitt auf. Er ist dazu ausgebildet, in den Markkanal eines röhrenartigen Knochens (nicht dargestellt), insbesondere eines Femurs, eingesetzt zu werden. Seiner Funktion als Spacer entsprechend wird er hierbei in denjenigen Freiraum eingesetzt, der durch die Entfernung einer zuvor implantierten Femurkomponente einer mit dem Acetabulum zusammenwirkenden Hüftprothese entstanden ist.

An den Schaft 10 schließt sich der Hals 2 an, wobei in dem dargestellten Ausführungsbeispiel dazwischen eine Auflageplatte 12 vorgesehen ist. Sie dient zur Aufnahme von in Längsrichtung des Schafts 1 wirkenden Kräften und leitet sie weiter auf eine Resektionsfläche an den Knochen, in den der Schaft eingesetzt ist.

Weiter schließt sich an den Hals 2 auf der dem Schaft 10 abgewandten Seite ein Gelenkstück 3 an. Das Gelenkstück 3 ist in dem dargestellten Ausführungsbeispiel von halbkugeliger Gestalt. Sein Pol liegt etwa in der Verlängerung der Mittelachse des Halses 2. Die Weite des Halses 2 nimmt zu dem Gelenkkopf 3 hin zu, ohne jedoch dessen volle Weite zu erreichen, sodass sich ein noch leicht stufiger Übergang ergibt.

Der Schaft 10, der Hals 2 sowie das Gelenkstück 3 sind vorzugsweise aus demselben Material gefertigt. Hierzu ist ein Kunststoffmaterial vorgesehen. Zweckmäßigerweise handelt es sich um Knochenzement, insbesondere Polymethylmethacrylat (PMMA). Dieses bietet den Vorteil guter Biokompatibilität und verbindet dies mit guter Formbarkeit, insbesondere prä- oder intraoperativ. Das Kunststoffmaterial ist mit einem Medikamentenwirkstoff (durch Punktierung 8 dargestellt) versehen, insbesondere einem Antibiotikum oder einem anderen Wirkstoff zur Bekämpfung von Entzündungen.

Erfindungsgemäß ist auf den halbkugeligen Gelenkkopf 3 eine Überkappung 4 aufgesetzt. Die Überkappung 4 besteht aus einem metallischen Material, insbesondere rostfreiem Edelstahl. Dies ist preisgünstig herzustellen, gut verarbeitbar, weist eine ausreichend hohe Biokompatibilität und günstige Reibungseigenschaften auf. Die Überkappung 4 ist ebenfalls sphärisch gestaltet, nämlich halbkugelförmig. Hierbei ist die Innenweite so bemessen, dass die Überkappung 4 straff auf dem Gelenkkopf 3 sitzt. Damit ummantelt die Überkappung 4 die sphärische Außenseite des Gelenkstücks 3 nach außen hin.

Die Überkappung 4 verhindert so einen unmittelbaren Kontakt des Kunststoffmaterials, welches das Gelenkstück 3 bildet, mit dem entsprechenden Gegenpart des Gelenks, also mit der Gelenkstückaufnahme 9. Im Falle einer Hüftprothese handelt es sich bei der Gelenkstückaufnahme 9 um das Acetabulum. Damit wird das Entstehen von Abrieb von dem Kunststoffmaterial bei Bewegung des Schafts 1 verhindert. Da die Überkappung 4 straff auf dem Gelenkkopf sitzt, bewegt sie sich nicht relativ zu diesem, so dass auch an dieser Grenzfläche kein Abrieb entstehen kann. Es wird somit wirkungsvoll verhindert, dass bei der Bewegung des Gelenks für den Patienten äußerst schädlicher Abrieb entsteht.

Die Überkappung 4 weist aufgrund ihrer Bauweise aus metallischem Material ein günstiges Reibungsverhalten in Bezug auf die Gelenkaufnahme 9 auf. Damit wird auch an dieser Stelle das Entstehen von Abrieb wirksam verhindert.

Wesentlich für eine effektive Funktion der Erfindung ist es, dass die Überkappung 4 sich nicht relativ zu dem Gelenkkopf 3 bewegt. Dazu ist eine Rotationssicherung 5 vorgesehen. Sie umfasst einen an der Innenseite der Überkappung 4 angeordneten metallischen Stift 51, der vorzugsweise im montierten Zustand in eine komplementär geformte Vertiefung 52 an dem Gelenkstück 3 eingreift. Auf diese Weise wird eine Sicherung der Überkappung 4 vor unerwünschter Rotation erreicht.

Durch den festen Sitz der Überkappung 4 in Kombination mit der Rotationssicherung 5 ist in vielen Fällen auch eine ausreichende Sicherheit gegenüber einem unerwünschten Abheben der Überkappung 4 von dem Gelenkkopf 3 realisiert. Optional kann aber auch eine gesonderte Dislokationssperre 6 vorgesehen sein. Sie umfasst in dem dargestellten Ausführungsbeispiel zwei am Rand der Überkappung 4 diametral einander gegenüberliegend angeordnete Zungen 61, 61'. Sie sind so ausgestaltet, dass sie im aufgesetzten Zustand in entsprechende komplementäre Aussparungen 62 am Gelenkkopf 3 eingreifen und dort eine formschlüssige Verriegelung bewirken. Die Zungen 61, 61' bestehen aus elastischem Material, sodass sie zum Aufsetzen der Überkappung 4 auf das Gelenkstück 3 elastisch nach außen gedrängt werden, und beim Erreichen der korrekten Position in die Aussparungen 62 einschnappen. Auf diese Weise wird die

Überkappung 4 in ihrer Positionierung relativ zu dem Gelenckopf 3 gesichert und ein Abheben in axialer Richtung vermieden.

In dem Schaft 10 ist eine innenliegende Tragstruktur 7 vorgesehen. Sie führt von dem Endbereich des Schafts 1 etwa entlang der Mittelachse des Schafts bis in den Hals 2, und ggf. auch darüber hinaus bis in das Gelenkstück 3 hinein. Sie bewirkt eine mechanische Aussteifung des Schafts 10, Hals 2 und ggf. des Gelenkstücks 3.

Bei einer Variante ist die Überkappung 4' mit innenliegenden Rippen 48 versehen (s. Fig. 3a). Sie bewirken eine zusätzliche Befestigung der Überkappung 4' auf dem Gelenkkopf 3. Sie stellen damit eine zusätzliche Sicherung gegenüber unerwünschter Verdrehung oder Dislokation dar.

Bei einer weiteren Variante ist die Überkappung 4" mit einer Vielzahl von Durchgangsöffnungen 44 versehen (s. Fig. 3b). Sie erleichtern einen Durchtritt des Medikamentenwirkstoffs 8 aus dem Kunststoffmaterial, das das Gelenkstück 3 bildet. Die Durchgangsöffnungen 44 können gleichmäßig angeordnet sein oder mit unterschiedlicher Anordnungsdichte, um so die Einwirkung des Medikamentenwirkstoffs 8 räumlich zu differenzieren. Besonders bevorzugt ist es, die Durchgangsöffnungen 44 nur in einem nicht-lasttragenden Bereich 63 anzuordnen.

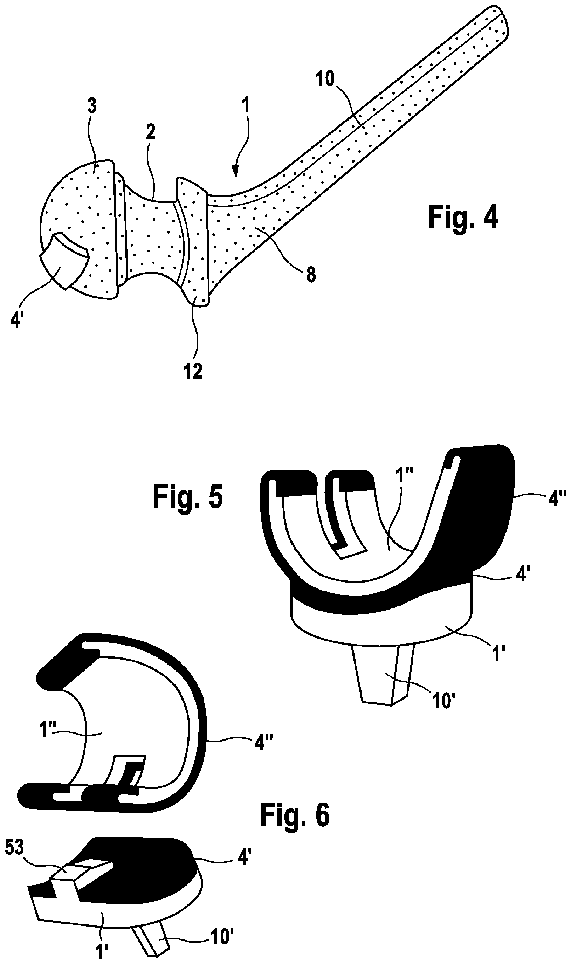

Ein zweites Ausführungsbeispiel ist in Fig. 4 dargestellt. Es basiert auf dem in Fig. 1 dargestellten ersten Ausführungsbeispiel und identische Elemente sind mit denselben Bezugsziffern bezeichnet. Im Unterschied zu dem ersten Ausführungsbeispiel ist die Überkappung nicht halbkugelförmig ausgebildet, so dass sie das Gelenkstück 3 nicht voll umfasst. Vielmehr besteht die Überkappung bei dem zweiten Ausführungsbeispiel aus einem konvexen Kugeloberflächensegment 4*, das an einer vorbestimmten Position auf dem Gelenkkopf 3 angeordnet ist. Hier ist die vorbestimmte Position so gewählt, dass das Segment an der Stelle angeordnet ist, wo (bei stehendem Patienten) die Hauptlast von der Gelenkstückaufnahme auf den Spacer übertragen wird. Funktional wird damit wie bei dem ersten Ausführungsbeispiel gemäß Fig. 1 ein Schutz gegenüber verschleißbedingtem Abrieb erreicht. Dies gilt jedoch nur für einen verhältnismäßig kleinen Bewegungsbereich. Dem steht als Vorteil gegenüber, dass bei der zweiten Ausführungsform gerade im Bereich des Gelenkstücks 3 die Versorgung des umliegenden Gewebes mit dem Medikamentenwirkstoff 8 deutlich verbessert ist.

Ein drittes Ausführungsbeispiel ist in den Fig. 5 und 6 dargestellt. Es ist ausgeführt als Spacer für eine Knieprothese. Vorgesehen sind zwei Komponenten, eine Femurkomponente und eine Tibiakomponente. Jeder der beiden Komponenten weist einen Hauptkörper 1', 1" auf. Im Fall der Tibiakomponente ist deren Hauptkörper 1' mit einem kurzen Schaft 10' versehen, der zur Verankerung in einem Markkanal am proximalen Ende einer Tibia ausgebildet ist. Wie insbesondere in der Explosionsansicht gemäß Fig. 6 gut zu erkennen ist, ist auf der dem Schaft 10' abgewandten Seite des Hauptkörpers 1' eine Überkappung 4' angeordnet, die als leicht konkav gebogenes Plateau ausgeführt ist. Hierbei fungiert ein Mittelsteg 53 als Verdrehsicherung.

Das komplementär damit zusammenwirkende Gegenstück ist die Femurkomponente. Sie umfasst einen etwa halbschalenartig geformten Hauptkörper 1" mit zwei kondylenartigen Fortsätzen an einer seiner Längsseiten. Die konvexe Außenfläche des halbschalenartig geformten Hauptkörpers 1" ist mit einer gleichfalls halbschalenartig geformten Überkappung 4" versehen. Hierbei gewährleistet die halbschalenartige Form der Überkappung 4" eine verdrehsichere Anordnung. An ihren Enden umgreift sie jeweils die längsseitige Berandung des Hauptkörpers 1" und ist somit gegenüber Dislokation gesichert.

Im montierten Zustand, wie in Fig. 5 dargestellt, wirken die Femurkomponente und die Tibiakomponente miteinander zusammen. Hierbei ist vorzugsweise eine der beiden Überkappungen 4', 4" aus einem metallischen Material und die andere aus einem verschleißfesten Kunststoffmaterial gefertigt. Es ergibt sich so ein Spacer mit einer einwandfreien Artikulationsfunktion bei minimiertem Abrieb. Es sei angemerkt, dass zur Vereinfachung auch vorgesehen sein kann, dass eine der beiden Überkappungen 4', 4" (aber nicht beide) entfällt. Auch dann wird die erfindungsgemäße Minimierung des Abriebs erreicht.

## Patentansprüche

1. Spacer für eine Gelenkendoprothese, umfassend einen Hauptkörper (1) zur Befestigung an einem Knochen und ein Gelenkstück (3) an einem Ende des Hauptkörpers (1), wobei der Hauptkörper (1) ein Material umfasst, das zur Abgabe eines Medikamentenwirkstoffs (8) an umliegendes Gewebe ausgebildet ist, und das Gelenkstück (3) zur beweglichen Interaktion mit einer Gelenkstückaufnahme (9) ausgebildet ist, wobei eine gesonderte Überkappung (4) des Gelenkstücks (3) vorgesehen ist, die aus einem verschleißfesten Material besteht und verdrehfest auf dem Gelenkkopf (3) gehaltert ist,
**dadurch gekennzeichnet, dass**
die Überkappung (4) eine Mehrzahl von nur in einem nicht-lasttragenden Bereich der Überkappung (4) angeordneten Durchgangsöffnungen (44) aufweist.

2. Spacer nach Anspruch 1, **dadurch gekennzeichnet, dass** das verschleißfeste Material ein gleitgünstiges Material ist, insbesondere Edelstahl, CoCrMo, Titan, hochfestes Kunststoffmaterial (Polyetheretherketon - PEEK) oder andere biokompatible Materialien.

3. Spacer nach einem der vorangehenden Ansprüche, **dadurch gekennzeichnet, dass** die Überkappung (4) an ihrer der Gelenkstückaufnahme (9) zugewandten Außenseite geglättet ist zur reibungsarmen Interaktion mit der Gelenkstückaufnahme (9) .

4. Spacer nach einem der vorangehenden Ansprüche, **dadurch gekennzeichnet, dass** Verankerungselemente (5) vorgesehen sind zur verdrehfesten Halterung der Überkappung (4) auf dem Gelenkkopf (3).

5. Spacer nach einem der vorangehenden Ansprüche, **dadurch gekennzeichnet, dass** die Überkappung (4) eine halbkugelförmige Gestalt aufweist.

6. Spacer nach einem der vorangehenden Ansprüche, **dadurch gekennzeichnet, dass** eine Dislokationssicherung an der Überkappung (4) vorgesehen ist, die ein Ablösen von dem Gelenkkopf (3) verhindert.

7. Spacer nach einem der vorangehenden Ansprüche, **dadurch gekennzeichnet, dass** eine Rotationssicherung (5) für die Überkappung (4) vorgesehen ist, die ein Verdrehen der Überkappung (4) relativ zu dem Gelenkkopf (3) verhindert.

8. Spacer nach einem der vorangehenden Ansprüche, **dadurch gekennzeichnet, dass** der Hauptkörper eine das Ende des Knochens umgreifende Gestalt aufweist.

9. Spacer nach einem der vorangehenden Ansprüche, **dadurch gekennzeichnet, dass** der Hauptkörper (1) einen Verankerungsschaft (10) umfasst, der vorzugsweise zum Einsetzen in einen Hohlraum des Knochens ausgebildet ist.

10. Spacer nach einem der vorangehenden Ansprüche, **dadurch gekennzeichnet, dass** eine innere Tragstruktur (7) im Schaft vorgesehen ist.

11. Spacer nach einem der vorangehenden Ansprüche, **dadurch gekennzeichnet, dass** die Überkappung (4) ein konvexes Kugeloberflächensegment (4*) ist.

12. Spacer nach einem der vorangehenden Ansprüche, **dadurch gekennzeichnet, dass** die Überkappung als Vollmaterialstück ausgeführt ist und als Gelenkstück (3) fungiert.

## Claims

1. Spacer for a joint endoprosthesis, comprising a main body (1), for attachment to a bone, and a joint piece (3) at one end of the main body (1), wherein the main body (1) comprises a material designed to deliver a medical active substance (8) to surrounding tissue, and the joint piece (3) is designed for movable interaction with a joint piece receiver (9), wherein a separate overcap (4) of the joint piece (3) is provided, which is made of a wear-resistant material and is mounted on the joint head (3) in a manner secure against rotation, **characterized in that** the overcap (4) has a plurality of through-openings (44) arranged only in a non-load-bearing region of the overcap (4).

2. Spacer according to Claim 1, **characterized in that** the wear-resistant material is a material that promotes sliding, in particular stainless steel, CoCrMo, titanium, high-strength plastic material (polyether ether ketone - PEEK), or other biocompatible materials.

3. Spacer according to one of the preceding claims, **characterized in that** the overcap (4), on its outer face directed towards the joint piece receiver (9), is smoothed for low-friction interaction with the joint piece receiver (9).

4. Spacer according to one of the preceding claims, **characterized in that** anchoring elements (5) are provided for mounting the overcap (4) on the joint head (3) in a manner secure against rotation.

5. Spacer according to one of the preceding claims, **characterized in that** the overcap (4) has a hemispherical shape.

6. Spacer according to one of the preceding claims, **characterized in that** a means of securing against dislocation is provided on the overcap (4) and prevents detachment from the joint head (3).

7. Spacer according to one of the preceding claims, **characterized in that** a means (5) of securing against rotation is provided for the overcap (4) and prevents a rotation of the overcap (4) relative to the joint head (3) .

8. Spacer according to one of the preceding claims, **characterized in that** the main body has a shape engaging around the end of the bone.

9. Spacer according to one of the preceding claims, **characterized in that** the main body (1) comprises an anchoring shaft (10), which is preferably designed for insertion into a cavity of the bone.

10. Spacer according to one of the preceding claims, **characterized in that** an inner support structure (7) is provided in the shaft.

11. Spacer according to one of the preceding claims, **characterized in that** the overcap (4) is a convex spherical surface segment (4*).

12. Spacer according to one of the preceding claims, **characterized in that** the overcap is designed as a piece of solid material and functions as a joint piece (3) .

## Revendications

1. Espaceur pour une endoprothèse d'articulation, comprenant un corps principal (1) pour la fixation à un os, et une pièce d'articulation (3) à une extrémité du corps principal (1), le corps principal (1) comprenant un matériau qui est prévu pour délivrer une substance active médicamenteuse (8) à un tissu environnant, la pièce d'articulation (3) étant réalisée de manière à interagir de manière mobile avec un logement de pièce d'articulation (9), une coiffe séparée (4) de la pièce d'articulation (3) étant prévue, laquelle se compose d'un matériau résistant à l'usure et est retenue de manière solidaire en rotation sur la tête d'articulation (3),
**caractérisé en ce que**
la coiffe (4) présente une pluralité d'ouvertures de passage (44) disposées seulement dans une région non porteuse de charge de la coiffe (4).

2. Espaceur selon la revendication 1, **caractérisé en ce que** le matériau résistant à l'usure est un matériau favorisant le glissement, en particulier de l'acier inoxydable, du CoCrMo, du titane, une matière plastique hautement résistante (polyéther éther cétone - PEEK) ou d'autres matériaux biocompatibles.

3. Espaceur selon l'une quelconque des revendications précédentes, **caractérisé en ce que** la coiffe (4) est lissée au niveau de son côté extérieur tourné vers le logement de pièce d'articulation (9) en vue de l'interaction à faible frottement avec le logement de pièce d'articulation (9).

4. Espaceur selon l'une quelconque des revendications précédentes, **caractérisé en ce que** des éléments d'ancrage (5) sont prévus pour retenir de manière solidaire en rotation la coiffe (4) sur la tête d'articulation (3).

5. Espaceur selon l'une quelconque des revendications précédentes, **caractérisé en ce que** la coiffe (4) présente une forme hémisphérique.

6. Espaceur selon l'une quelconque des revendications précédentes, **caractérisé en ce qu'**une protection contre la dislocation est prévue au niveau de la coiffe (4), laquelle empêche qu'elle ne se détache de la tête d'articulation (3).

7. Espaceur selon l'une quelconque des revendications précédentes, **caractérisé en ce qu'**une protection contre la rotation (5) est prévue pour la coiffe (4), laquelle empêche une rotation de la coiffe (4) par rapport à la tête d'articulation (3).

8. Espaceur selon l'une quelconque des revendications précédentes, **caractérisé en ce que** le corps principal présente une forme venant en prise autour de l'extrémité de l'os.

9. Espaceur selon l'une quelconque des revendications précédentes, **caractérisé en ce que** le corps principal (1) comprend une tige d'ancrage (10) qui est de préférence réalisée de manière à être insérée dans une cavité de l'os.

10. Espaceur selon l'une quelconque des revendications précédentes, **caractérisé en ce qu'**une structure porteuse intérieure (7) est prévue dans la tige.

11. Espaceur selon l'une quelconque des revendications précédentes, **caractérisé en ce que** la coiffe (4) est un segment de surface sphérique convexe (4*).

12. Espaceur selon l'une quelconque des revendications précédentes, **caractérisé en ce que** la coiffe est réalisée sous forme de pièce en matériau massif et sert de pièce d'articulation (3).
